# EUROPEAN PATENT APPLICATION

(11) **EP 1 947 176 A1**
(43) Date of publication of application: **23.07.2008**
(21) Application number: 06823269.3
(22) Date of filing: 09.11.2006
(51) Int. Cl.: C12N 9/96, C07K 1/00, C07K 16/00, G01N 33/531

(54) **METHOD FOR STABILIZATION OF BIOLOGICAL MOLECULE AND COMPOSITION**

(30) Priority: 11.11.2005 JP 2005327590
(71) Applicant: Toyo Boseki Kabushiki Kaisha, Osaka-shi, Osaka 530-8230 (JP); SEIREN CO., LTD., Fukui-shi, Fukui 918-8560 (JP)
(72) Inventor: KISHIMOTO, Takahide, Tsuruga-shi, Fukui 914-0047 (JP); KAJITANI, Kayoko, Tsuruga-shi, Fukui 914-0047 (JP); KAJITANI, Kazuo, Tsuruga-shi, Fukui 914-0047 (JP); YAMADA, Hideyuki, Fukui-shi, Fukui 918-8560 (JP); SASAKI, Masahiro, Fukui-shi, Fukui 918-8560 (JP)
(74) Representative: Vossius & Partner
(86) International application number: PCT/JP2006/322382
(87) International publication number: WO 2007/055284

(57) **Abstract**

The object is to provide a method for stabilization of a biological molecule and a composition, specifically a method for stabilization of an enzyme or a labeled antibody for use in a clinical diagnosis and a composition. Thus, disclosed is a method for stabilization of a biological molecule which is **characterized by** allowing (a) the biological molecule and (b) sericin and/or a hydrolysate or equivalence thereof to coexist with each other. Also disclosed is a composition having a biological molecule stabilized therein, which is **characterized in that** the components (a) and (b) coexist with each other in the composition. Further disclosed is a composition for stabilizing a biological molecule, which comprises sericin and/or a hydrolysate or equivalence thereof.

## Description

### Technical Field

The present invention relates to a method for stabilizing a biological molecule and a composition comprising a biological molecule, in particular, a method for stabilizing an enzyme or a labeled antibody used in clinical diagnosis and a composition comprising the enzyme or the labeled antibody.

### Background Art

Enzymes, and labeled antibodies produced by modifying antibodies with enzymes using various compounds are applied to various uses because of their high substrate specificity and ease of handling. For example, they are used for producing analysis reagents for molecular biological use, analysis reagents for biochemical use, extracorporeal diagnostic agents, liquid extracorporeal diagnostic agents, dried extracorporeal diagnostic agents in the form of chips or slits, enzyme sensors or enzyme electrodes, drugs, foods, beverages and the like. Enzymes to be used in producing the above-described compositions may be labeled with labeling compounds such as dye, biotin and avidin, modified with various compounds, or conjugated with antibodies or antigens.

In order to maintain the efficacy of such compositions containing enzymes or labeled antibodies over a long period of time, it is important to stably retain the activity of the enzymes or labeled antibodies. For example, when the activity of an enzyme in a reagent composition is reduced with time, an excess amount of the enzyme may be added beforehand to the composition, depending on the desired effective term of the reagent and the activity-lowering rate of the enzyme. In many cases, however, such a means does not fundamentally solve the problem and the cost for an enzyme or a labeled antibody to be used is increased.

Thus, stabilization of a composition has been attempted by using various methods comprising adding a substrate, a coenzyme, a salt, an ion, a saccharide, a sugar alcohol, an amino acid, a fatty acid ester, a protein such as bovine serum albumin or gelatin hydrolysate or the like to the composition (see, for example, Patent Documents 1 to 5).

Patent Document 1: JP-A 2004-141162
Patent Document 2: Japanese Patent No. 3685815
Patent Document 3: Japanese Patent No. 3696267
Patent Document 4: JP-A 2005-114368
Patent Document 5: JP-A 10-279594

### Disclosure of Invention

### Problem to be solved by the Invention

However, in the case of using a coenzyme as a stabilizing agent, a coenzyme is generally expensive and, moreover, is expected to have an effect on only a specific biological molecule. In the case of using a high molecule, a salt or a certain amino acid in a solution as a stabilizing agent, it can not be added in a sufficient amount due to its low solubility or deposition during storage in some cases. In particular, in the case of adding a saccharide or an amino acid as a stabilizing agent to a diagnostic agent, the additive may be unexpectedly reacted with a contaminating substance present in a reagent system or contained in an enzyme or labeled antibody. Further, in the case of using an animal-derived ingredient such as bovine serum albumin as a stabilizing agent, it is necessary to pay careful attention to the risk of staining, bovine spongiform encephalopathy (BSE) or the like.

An objective of the present invention is to provide a method of effectively stabilizing a biological molecule such as an enzyme or a labeled antibody, and a composition comprising a biological molecule in which the stability of the biological molecule is maintained over a long period of time by the method.

### Means for Solving the Problem

The present inventors studied in various ways in order to attain the above-described objective. As a result, they found that a biological molecule could be effectively stabilized by allowing the biological molecule and sericin and/or a hydrolysate or equivalent thereof to coexist with each other. Thus, the present invention was completed.

The present invention provides:
(1) a method for stabilizing a biological molecule, which comprises allowing the following (a) and (b) to coexist with each other:
   (a) a biological molecule; and
   (b) sericin and/or a hydrolysate or equivalent thereof;
(2) the method according to the above (1), wherein the biological molecule is present in a solution;
(3) the method according to the above (1), wherein the biological molecule is present in a lyophilized composition;
(4) the method according to the above (1), wherein the sericin and/or a hydrolysate thereof is derived from naturally-occurring sericin extracted from cocoon filaments or raw silk;
(5) the method according to the above (1), wherein the equivalent of sericin is obtained by a genetic engineering technique;
(6) the method according to the above (1), wherein the biological molecule is a protein;
(7) the method according to the above (1), wherein the biological molecule is an enzyme;
(8) the method according to the above (1), wherein the biological molecule is a labeled antibody;
(9) a composition comprising a stabilized biological molecule, wherein the following (a) and (b) coexist with each other in the composition:
   (a) a biological molecule; and
   (b) sericin and/or a hydrolysate or equivalent thereof;
(10) the liquid composition according to the above (9), wherein the biological molecule is present in a solution;
(11) the composition according to the above (9), wherein the biological molecule is present in a lyophilized composition;
(12) the composition according to the above (9), wherein the sericin and/or a hydrolysate thereof is derived from naturally-occurring sericin extracted from cocoon filaments or raw silk;
(13) the composition according to the above (9), wherein the equivalent of sericin is obtained by a genetic engineering technique;
(14) the composition according to the above (9), wherein the biological molecule is a protein;
(15) the composition according to the above (9), wherein the biological molecule is an enzyme;
(16) the composition according to the above (9), wherein the biological molecule is a labeled antibody;
(17) a method for producing a composition comprising a stabilized biological molecule, which comprises a step of allowing sericin and/or a hydrolysate or equivalent thereof to coexist with a biological molecule;
(18) a composition for stabilizing a biological molecule, comprising sericin and/or a hydrolysate or equivalent thereof;
(19) a kit for diagnosis which comprises the composition comprising a stabilized biological molecule according to the above (9); and
(20) a biosensor which comprises the composition comprising a stabilized biological molecule according to the above (9).

### Effect of the Invention

According to the present invention, it is possible to enhance the stability of a biological molecule by using sericin and/or a hydrolysate or equivalent of sericin regardless of whether the biological molecule is in a liquid state or in a dried state, and thereby, the efficacy of a composition comprising the biological molecule can be maintained over a long period of time.
According to the method for stabilizing a biological molecule of the present invention, it is possible to enhance the stability of an enzyme or a labeled antibody regardless of whether it is in a liquid state or in a dried state, and thereby, the efficacy of a composition comprising the biological molecule can be maintained over a long period of time.

### Best Mode for Carrying Out the Invention

Hereinafter, the present invention is explained in detail.
The term "stabilization", as used herein, refers to such a state that the remaining function which a biological molecule retains after storage under the condition (a) is greater than the remaining function which the biological molecule retains after storage under the condition (b), wherein the condition (a) is keeping of the biological molecule in the presence of a certain substance for a certain period and the condition (b) is keeping of the biological molecule in the absence of the certain substance for the certain period.
For example, the term "stabilization" refers to such a state that the remaining activity of a biological molecule such as an enzyme or a labeled antibody contained in an aqueous solution after storage under the condition (a) is greater than that after storage under the condition (b), wherein the condition (a) is keeping of the aqueous solution containing the biological molecule and a certain substance at a suitable temperature for a certain period and the condition (b) is keeping of the aqueous solution containing the biological molecule and not containing the certain substance at the suitable temperature for the certain period.
Further, for example, the term "stabilization" refers to such a state that the remaining activity of a biological molecule such as an enzyme or a labeled antibody contained- in a dried composition after storage under the condition (a) is greater than that after storage under the condition (b), wherein the condition (a) is keeping of the dried composition containing the biological molecule and a certain substance at a suitable temperature for a certain period and the condition (b) is keeping of the dried composition containing the biological molecule and not containing the certain substance at the suitable temperature for the certain period.
Evaluation of stabilization can be performed, for example, by measuring a decrease of the remaining function with time in the presence of a certain substance [the condition (a)] and in the absence of the certain substance [the condition (b)] and then comparing half-lives in the respective conditions.
A condition for "storage at a suitable temperature for a certain period" is not particularly limited as long as it produces a difference in the remaining activity between the conditions (a) and (b). Preferably, a condition for an acceleration (severity) test intended to determine long-term storage stability of a biological molecule in a diagnostic reagent or the like is selected. Specific examples of such a condition include "storage at 40°C for 7 days" and "storage at 52°C for 1 hour". If given sufficient time, a condition of storage for 6 months or longer under refrigeration at 2°C to 10°C, which is a temperature range generally used for long storage of a diagnostic agent containing a biological molecule or the like, may be selected.

One embodiment of the present invention is a method for increasing the remaining activity of an enzyme after storage in 50mM PIPES-NaOH buffer (pH 7.0) at 25°C for 2 months which comprises adding sericin, as compared with that after storage without sericin.
Another embodiment of the present invention is a method for increasing the remaining activity of an enzyme after storage in 50mM PIPES-NaOH buffer (pH 7.0) containing 1 g/L of Triton X-100 at 40°C for 14 days which comprises adding sericin, as compared with that after storage without sericin.
Further another embodiment of the present invention is a method for increasing the remaining activity of an enzyme after storage in 50mM potassium phosphate buffer (pH 7.0) at 52°C for 1 hour which comprises adding sericin, as compared with that after storage without sericin.
Further another embodiment of the present invention is a method for increasing the remaining activity of an enzyme after storage in 50mM Tris-HCl buffer (pH 8.0) containing 1 g/L of Triton X-100 at 9°C for 24 hours which comprises adding sericin, as compared with that after storage without sericin.
Further another embodiment of the present invention is a method for increasing the remaining activity of an enzyme after storage in 50mM PIPES-NaOH buffer (pH 7.0) containing 1 g/L of Triton X-100 at 25°C for 14 days which comprises adding sericin, as compared with that after storage without sericin.
Further another embodiment of the present invention is a method for increasing the remaining activity of an enzyme after storage in 50mM potassium phosphate buffer (pH 7.0) containing 1 g/L of Triton X-100 at 25°C for 14 days which comprises adding sericin, as compared with that after storage without sericin.
Further another embodiment of the present invention is a method for increasing the remaining activity of an enzyme after storage in 50mM PIPES-NaOH buffer (pH 7.0) containing 0.5 g/L of sodium azide at 25°C for 14 days which comprises adding sericin, as compared with that after storage without sericin.
Further another embodiment of the present invention is a method for increasing the remaining activity of an enzyme after storage in a lyophilized powder form at 37°C for 14 days which comprises adding sericin, as compared with that after storage without sericin.
Further another embodiment of the present invention is a method for increasing the remaining activity of a labeled antibody after storage in 20mM MOPS buffer (pH 7.0) at 4°C for 3 days which comprises adding sericin, as compared with that after storage without sericin.
Further another embodiment of the present invention is a method for increasing the remaining activity of a labeld antibody after storage in 0.1M Tris-HCl buffer (pH 7.4) containing 1 mM magnesium chloride, 0.1 mM zinc chloride and 1 g/L of sodium azide at 4°C for 3 days which comprises adding sericin, as compared with that after storage without sericin.
In the above-described embodiments, a sericin hydrolysate or a sericin equivalent may be used in place of sericin.

Sericin is a noncrystalline naturally-occurring protein present in cocoon filaments or raw silk. In particular, the "sericin" as used herein refers to sericin extracted in a non-hydrolyzed state from cocoon filaments or raw silk.
A gene of sericin has generally a size of 2.6 kbp to 10.6 kbp. Some genes of sericin have been determined and they are shown, for example, in The Journal of Biological Chemistry, 257: 15192-15199 (1982). The sericin used in the present invention is a protein produced from a gene having such a sequence. An example of the sericin used in the present invention is a protein substantially consisting of an amino acid sequence set forth in SEQ ID NO: 2 as its full-length sequence. Typically, the amino acid sequence of SEQ ID NO: 2 is composed of an essential region consisting of a 38-amino acid sequence set forth in SEQ ID NO: 1 and a nonessential region other than the essential region, and it comprises the essential region as a repeat sequence. For example, sericin consisting of an amino acid sequence set forth in SEQ ID NO: 2 comprises 12 repeated essential regions.
The phrase "substantially consisting of an amino acid sequence set forth in SEQ ID NO: 2" concerning sericin, as used herein, means that the amino acid sequence (SEQ ID NO: 2) may have deletion, substitution, insertion or addition of one or more, preferably 1 to 2000, more preferably 1 to 500, further more preferably 1 to 300 amino acid residues as long as the sericin has the ability to stabilize a biological molecule. In this case, it is preferable that deletion, substitution, insertion or addition of amino acid residues is placed in a region other than the essential region. In the case of an amino acid residue in the essential region, conservative substitution is preferred. In the essential region, one or more, preferably 1 to 50, more preferably 1 to 20 amino acid residues of the amino acid sequence may be conservatively substituted as long as the sericin has the ability to stabilize a biological molecule. The term "conservative substitution" means that one or more amino acid residues are substituted by other amino acid residues having chemically similar properties without substantial alteration of the activity of a protein. Examples of conservative substitution include substitution of a hydrophobic residue by another hydrophobic residue, substitution of a polar residue by another polar residue having the same charge, substitution of an aromatic amino acid by another aromatic amino acid, and the like. Such an amino acid functionally similar to each amino acid is known in the art.
In the present invention, a hydrolysate of sericin can be used in place of or in combination with sericin in a non-hydrolyzed state as long as it has the ability to stabilize a biological molecule.
Sericin and a sericin hydrolysate can be obtained by extraction from cocoon filaments or raw silk according to a known method disclosed in W02002/086133. Specifically, sericin in a non-hydrolyzed state can be obtained as a highly pure protein with purity of 90% or more, for example, by a method as described below.
First, sericin present in cocoon filaments or raw silk is extracted with water by a treatment of cocoon filaments or raw silk with water, preferably hot water at about 80 to 100°C, to obtain an aqueous solution of sericin. Then, the aqueous solution of sericin can be subjected to a separation/purification treatment, for example any one of the following methods (1) to (3), to recover the desired sericin.
(1) The aqueous solution of sericin is adjusted to pH 3 to 5 with addition of organic or inorganic acid. Then, an organic or inorganic flocculant is added to the solution to allow sericin to be deposited. The deposition can be separated by filtration and then dried to obtain sericin in a solid state.
(2) The aqueous solution of sericin is mixed with an water-miscible solvent such as methanol, ethanol or dioxane to allow sericin to be deposited. The deposition can be separated by filtration and then dried to obtain sericin in a solid state.
(3) The aqueous solution of sericin is subjected to a given filtration treatment with an ultrafiltration membrane or a reverse osmosis membrane, and then dried to obtain sericin in a powdery state, as described in JP-A 4-202435.
A sericin hydrolysate can be obtained as a highly pure protein with purity of 90% or more, for example, by a method as described below.
First, sericin present in cocoon filaments or raw silk is extracted with water by a treatment of cocoon filaments or raw silk with water, preferably hot water at about 80 to 100°C, to obtain an aqueous solution of sericin. At this time, sericin is partially hydrolyzed using electrolyzed water, acid, alkali or enzyme in combination. The resulting aqueous solution of a sericin hydrolysate can be subjected to a separation/purification treatment, for example any one of the above-described methods (1) to (3), to recover the desired sericin hydrolysate.
The sericin or sericin hydrolysate thus obtained has usually a molecular weight of 500 to 500,000. Although any sericin or sericin hydrolysate having a molecular weight within the range can be used as long as it has the ability to stabilize a biological molecule, a sericin hydrolysate having an average molecular weight of 5,000 to 100,000, especially 10,000 to 50,000 is preferred in view of handling.

The term "equivalent thereof" (hereinafter, also referred to as "sericin equivalent") of the "sericin and/or a hydrolysate or equivalent thereof" used in the stabilizing method of the present invention refers to a polypeptide which comprises at least the above-described essential region of naturally-occurring sericin (i.e., the 38-amino acid sequence set forth in SEQ ID NO: 1) and is produced artificially (i.e., by chemical synthesis or genetic engineering technique). Thus, the artificially-produced sericin equivalent can be a polypeptide consisting of the amino acid sequence identical to that of sericin or a sericin hydrolysate derived from natural products. Further, examples of a sericin equivalent include products synthesized utilizing a part of naturally-occurring sericin or a sericin hydrolysate as a base.
In the present invention, the "equivalent thereof" can be used in place of the "sericin and/or a hydrolysate thereof" derived from natural products as long as it has the ability to stabilize a biological molecule.
A sericin equivalent preferably comprises the essential region consisting of 38-amino acid sequence of SEQ ID NO: 1 as a repeat sequence which is repeated more than once. The ability to stabilize a biological molecule can be enhanced by using a polypeptide having the repeat sequence. A sericin equivalent comprises preferably at least one essential region, more preferably at least two essential regions per 100 amino acid residues in its amino acid sequence. It is preferable that the proportion of the essential region present in a sericin equivalent is higher. A sericin equivalent comprising such a high proportion of the repeat sequence can stably show the ability to stabilize a biological molecule even if the full-length amino acid residue number of the sericin equivalent is increased. Typically, the ability of a sericin equivalent is equivalent or superior to that of sericin or a sericin hydrolysate derived from natural products.
The full length of a sericin equivalent is not particularly limited as long as the sericin equivalent has the ability to stabilize a biological molecule. From the standpoint of production, in the full length of a sericin equivalent, the essential region is repeated preferably 2 to 8 times, more preferably 2 to 6 times, still more preferably 2 to 4 times.
The amino acid sequence of the essential region present in a sericin equivalent may has conservative substitution, for example, of one or more, preferably 1 to 5, more preferably 1 to 3 amino acid residues.
A sericin equivalent can be obtained according to a known method disclosed in WO2002/086133. For example, in the case of chemically synthesizing a sericin equivalent, a conventional polypeptide synthesis means such as solid-phase or liquid-phase synthesis according to the t-Boc method or the Fmoc method can be appropriately used. In the case of producing a sericin equivalent by a genetic engineering technique, if a DNA encoding a sericin equivalent can be purchased or produced, a host cell can be transformed with the DNA to allow the transformed cell to produce a sericin equivalent.
A sericin equivalent can be a fusion protein. Such a fusion protein can be produced by fusing a DNA encoding the above-described "sericin" or "sericin hydrolysate" with a DNA encoding a heterologous polypeptide to produce a DNA encoding a fusion protein and then expressing the DNA.

Sericin and/or a hydrolysate or equivalent thereof can be added, for example, at a concentration of 0.1 to 200 g/L, preferably 0.1 to 100 g/L, more preferably 0.2 to 20 g/L.

A biological molecule to be subjected to the stabilizing method of the present invention is not particularly limited. Examples of the biological molecule include protein (e.g., an enzyme, a labeled antibody and the like used for clinical diagnosis), nucleic acid, and compositions containing protein and/or nucleic acid.
The type of an enzyme to be used as the biological molecule is not particularly limited. Examples of the enzyme include peroxidase, lipase, protease, amylase, glucoamylase, ascorbate oxidase, β-glucosidase, uricase, pyruvate dehydrogenase, glycerol kinase, lactate dehydrogenase, glutamate dehydrogenase, catalase, cholesterol oxidase, glucose oxidase, glucose dehydrogenase, creatinine amidohydrolase (creatinine amide hydrolase), alkaline phosphatase, β-galactosidase and the like.

The enzyme to be used as the biological molecule can be prepared from a naturally-occurring source such as a microorganism, an animal or a plant, produced by a genetic engineering technique, or chemically synthesized. The enzyme may be obtained by modifying a wild-type enzyme using a protein engineering technique or the like.
The enzyme used in the present invention may be labeled with a dye or a labeling compound (e.g., biotin or avidin), modified with a compound, or conjugated with an antibody or an antigen.
Examples of a labeled antibody and a labeled antigen to be used as the biological molecule include, but not limited to, the following proteins, enzymes, hormones, antibodies, antigens and the like which are labeled with peroxidase, amylase, catalase, glucose oxidase, glucose dehydrogenase, alkaline phosphatase, β-galactosidase or the like: proteins such as mouse IgG, β2-microglobulin, carcinoembryonic antigen (CEA), immunoglobulin (IgG, IgA, IgM, IgD, IgE), C-reactive protein (CRP), α1-antitrypsin, α1-microglobulin, haptoglobin, transferrin, ceruloplasmin, ferritin, albumin, hemoglobin A1, hemoglobin A1C, myoglobin, myosin, dupan-2, a-fetoprotein (AFP), tissue polypeptide antigen (TPA), apolipoprotein A1, apolipoprotein E, rheumatoid factor, anti-streptolysin O (ASO), fibrin degradation product (FDP), fibrin degradation product D fraction (FDP-D), fibrin degradation product b.-D fraction (FDP-DDimer), fibrin degradation product E fraction (FDP-E), antithrombin-III (AT-III) and the like; enzymes such as amylase, prostatic acid phosphatase (PAP), neuron-specific enolase (NSE), fibrinogen, elastase, plasminogen, creatine kinase-myocardial band (CK-MB) and the like; hormones such as insulin, thyroid-stimulating hormone (TSH), 3,5,3'-triiodothyronine (T3), thyroxine (T4), adrenocorticotropic hormone (ACTH), growth hormone (GH), luteinizing hormone (LH) and the like; and antibodies and antigens for viruses responsible for various infections such as hepatitis B virus-associated antibody, hepatitis B virus-associated antigen, hepatitis C virus antibody, HTLV (adult T-cell leukemia virus) antibody, HIV (AIDS virus) antibody, chlamydia antibody, syphilis antibody, toxoplasma antibody and the like. The antibody includes a polyclonal antibody, a monoclonal antibody, a mixture of a monoclonal antibodies, an antibody fragment such as F(ab')2, Fab' or Fab which is fragmented by an enzymatic treatment or a genetic engineering technique.
The biological molecule as used in the present invention includes such a complex.

The state of the biological molecule is not particularly limited. The biological molecule may be present in a liquid state, a lyophilized state, a granular state, a state of being fixed on a support, or a state of coating a chip or the like.
The biological molecule may be also provided as a liquid composition, a lyophilized composition or the like which is a mixture with other substances.
Such a composition can be placed in a suitable container or mounted in a suitable device, and thereby it can take various forms including an analysis reagent for molecular biological use, an analysis reagent for biochemical use, an extracorporeal diagnostic agent, a liquid extracorporeal diagnostic agent, a dried extracorporeal diagnostic agent in the form of a chip or slit, an enzyme sensor, an enzyme electrode, a drug, a food, a beverage and the like.

In the present invention, a composition comprising the biological molecule may be further mixed with other substances for the purpose of stabilization, improvement of shape, and the like.
When a composition comprising the biological molecule is in a powdery state, it can contain, as a conventional stabilizer or shape-improving agent, a saccharide such as glucose, fructose, galactose, mannose, xylose, lactose, sucrose, raffinose, trehalose, cyclodextrin, pullulan, inulin, soluble starch or the like; a sugar alcohol such as glucitol, mannitol, inositol, xylitol or the like; an amino acid or amino acid salt such as glycine, alanine, serine, threonine, glutamic acid, aspartic acid, glutamine, asparagine, lysine, histidine or the like; a peptide such as glycylglycine, glycylglycylglycine or the like; an inorganic acid salt such as phosphate, borate, sulfate, Tris salt or the like; an organic acid or organic acid salt such as flavin, acetic acid, citric acid, malic acid, maleic acid, gluconic acid or the like; a protein such as gelatin, casein, albumin or the like; or a surfactant such as a cholic acid, a sugar ester of fatty acid, polyoxyethylene alkyl ether, polyethylene glycol alkyl ether, or the like.

A composition comprising the biological molecule in a liquid state includes not only a solution in which a biological molecule such as an enzyme protein is completely dissolved but also a dispersion in a liquid such as a suspension. An enzyme in a liquid state may be dissolved or suspended in water or in a phosphate buffer, an acetate buffer, a borate buffer, a citrate buffer, a Tris buffer or a Good's buffer (e.g., PIPES, MES, TES, MOPS, HEPES or the like). Such a liquid composition of an enzyme may contain a salt such as ammonium sulfate, ammonium phosphate, sodium chloride or potassium chloride. Such a liquid composition of an enzyme may further contain an alcohol such as ethanol, methanol or propanol, a polyol such as glycerol or ethylene glycol, or a surfactant such as an alkyl glucoside, a polyethylene glycol alkyl ether or a fatty acid alcohol ester. Optionally, an antibiotic of penicillin, cephem, aminoglycoside, macrolide, tetracyclin, new quinolone or the like, or a preservative such as azide, 1,1'-methylen-bis[3-(1-hydroxymethyl-2,4-dioximidazolidin-5-yl)-urea],2-methyl-3(2H)-isothiazolone-hydrochloride, 5-bromo-5-nitro-1,3-dioxane, 2-hydroxypyridine-N-oxide, 2-chloroacetamide or the like may be added to the liquid composition of an enzyme.

A composition comprising an enzyme and/or a labeled antibody, in accordance with the intended use, can contain a coenzyme such as NAD+, NADH, NADP+, NADPH, ATP, ADP, AMP, GTP, GMP, FAD, FMN, biotin, niacin, cobalamin, PQQ or the like; a salt of metal such as sodium, potassium, zinc, magnesium, calcium, lithium, copper, iron, manganese or the like; a thiol compound; a selenium compound; a salt such as nitrate, phosphate, sulfate, borate, Tris salt or the like; an amino acid or an amino acid salt; a sugar or a glycoside; or optionally, a phenolic or aniline Trinder's reagent and 4-aminoantipyrine as a coupler, a tetrazolium salt and an electron carrier such as phenazine methosulfate, or a dye such as a leuco reagent and a substrate.

A composition comprising a labeled antibody, in accordance with the intended use, may contain a nonspecific reaction-preventing agent. Examples of a nonspecific reaction-preventing agent include, but not limited to, an antibody of the same species as a labeled antibody and an enzyme of the same species as a labeled antibody. Examples of the antibody of the same species include mouse IgG, mouse IgM, mouse IgG polymer prepared by polymerization, goat IgG, sheep IgG, horse IgG, rat IgG and rabbit IgG. Although a body fluid containing such an antibody such as an animal serum or ascites fluid may be added as it is to the composition comprising a labeled antibody, it is preferable that the body fluid is added after a virus inactivation treatment, a complement inactivation treatment, a delipidation treatment or the like.

### Examples

Hereinafter, the present invention is specifically explained by means of Examples to which the present invention is not limit.

In the following Examples 1 to 21, a sericin hydrolysate was produced according to a method disclosed in Production Example 1 of WO2002/086133.
The details are as follows.

### Production Example 1

One kilogram of cocoons (made by domesticated silkworms (Bombyx mori)) were subjected to a hot-water treatment in 50 L of a 0.2% sodium carbonate aqueous solution (pH 11-12) at 95°C for 2 hours to extract a sericin hydrolysate. The resulting sericin hydrolysate extract was filtered through a filter having an average pore diameter of 0.2 µm to remove aggregates. The filtrate was then desalted through a reverse osmosis membrane to obtain a 0.2% transparent and colorless solution of a sericin hydrolysate in water.
The solution was concentrated with an evaporator to a concentration of about 2%, and then lyophilized to obtain 100 g of a sericin hydrolysate in a powder form with a purity of 90% or more and an average molecular weight of 20,000.

### Example 1

A reagent solution containing peroxidase (Toyobo, PEO-302) and a sericin hydrolysate as described below was stored at 25°C for 2 months or at 50°C for 7 days, and a remaining activity ratio (the ratio of activity after storage to activity immediately after preparation) was determined.

### Preparation of Reagent

Each reagent having the following composition was prepared.
PIPES-NaOH 50mM pH 7.0
Peroxidase (Toyobo, PEO-302) 5,000 U/L
Sericin hydrolysate 0.2 to 2 g/L

### Comparative Example 1

A reagent solution was prepared according to Example 1 except that the sericin hydrolysate was not added or replaced by 0.2 to 2 g/L of bovine serum albumin (Sigma, Fraction V). The reagent solution was stored under the same conditions as in Example 1 and a remaining activity ratio was determined.
As shown in Table I, it was found that peroxidase was stabilized by the sericin hydrolysate.

[Table 1]

**Table 1: Stabilization Effect of Sericin hydrolysate on Peroxidase solution**

| | Additive | Addition concentration (g/L) | Remaining ratio (%) | |
|---|---|---|---|---|
| | | | 25°C, 2 months | 50°C, 7 days |
| Example 1 | Sericin hydrolysate | 0.2 | 93 | 1 |
| | | 0.5 | 95 | 65 |
| | | 1 | 96 | 84 |
| | | 2 | 97 | 91 |
| Comparative Example 1 | Bovine serum albumin | 0.2 | 0 | 0 |
| | | 0.5 | 1 | 0 |
| | | 1 | 1 | 0 |
| | | 2 | 21 | 0 |
| | None | - | 60 | 0 |

### Example 2

To a reagent solution containing peroxidase and 2 g/L of a sericin hydrolysate as prepared in Example 1 was added 0.5 g/L, of sodium azide as a preservative to prepare a reagent solution. The reagent solution was then stored at 25°C for 14 days, and a remaining activity ratio (the ratio of activity after storage to activity immediately after preparation) was determined.

### Comparative Example 2

A reagent solution was prepared according to Example 2 except that the sericin hydrolysate was not added or replaced by 2 g/L of bovine serum albumin (Sigma, Fraction V). The reagent solution was stored under the same conditions as in Example 2 and a remaining activity ratio was determined.
As shown in Table 2, it was found that peroxidase was stabilized in the presence of the preservative by the sericin hydrolysate.

[Table 2]

**Table 2: Stabilization Effect of Sericin hydrolysate on Peroxidase solution**

| | Additive | Addition concentration (g/L) | Remaining ratio (%) |
|---|---|---|---|
| | | | 25°C, 14 days |
| Example 2 | Sericin hydrolysate | 2 | 80 |
| Comparative Example 2 | Bovine serum albumin | 2 | 37 |
| | None | - | 62 |

### Example 3

A reagent solution containing lipoprotein lipase (Toyobo, LPL-311) and a sericin hydrolysate as described below was stored at 25°C or 40°C for 14 days, and a remaining activity ratio (the ratio of activity after storage to activity immediately after preparation) was determined.

### Preparation of Reagent

Each reagent having the following composition was prepared.
PIPES-NaOH 50mM pH 7.0
Triton X-100 1 g/L
Lipoprotein lipase (Toyobo, LPL-311) 5,000 U/L
Sericin hydrolysate 0.2 to 2 g/L

### Comparative Example 3

A reagent solution was prepared according to Example 3 except that the sericin hydrolysate was not added or replaced by 0.2 to 2 g/L of bovine serum albumin (Sigma, Fraction V). The reagent solution was stored under the same conditions as in Example 3 and a remaining activity ratio was determined.
As shown in Table 3, it was found that lipoprotein lipase was stabilized by the sericin hydrolysate.

[Table 3]

**Table 3: Stabilization Effect of Sericin hydrolysate on Lipoprotein lipase solution**

| | Additive | Addition concentration (g/L) | Remaining ratio (%) | |
|---|---|---|---|---|
| | | | 25°C, 14 days | 40°C, 14 days |
| Example 3 | Sericin hydrolysate | 0.2 | 41 | 10 |
| | | 0.5 | 66 | 17 |
| | | 1 | 82 | 50 |
| | | 2 | 87 | 69 |
| Comparative Example 3 | Bovine serum albumin | 0.2 | 29 | 0 |
| | | 0.5 | 20 | 0 |
| | | 1 | 55 | 0 |
| | | 2 | 83 | 10 |
| | None | - | 18 | 0 |

### Example 4

A reagent solution containing glycerol kinase (Toyobo, GYK-301) and a sericin hydrolysate as described below was stored at 25°C for 14 days, and a remaining activity ratio (the ratio of activity after storage to activity immediately after preparation) was determined.

### Preparation of Reagent

A reagent having the following composition was prepared.
PIPES-NaOH 50mM pH 7.0
Triton X-100 1 g/L
Glycerol kinase (Toyobo, GYK-301) 2,000 U/L
Sericin hydrolysate 2 g/L

### Comparative Example 4

A reagent solution was prepared according to Example 4 except that the sericin hydrolysate was not added or replaced by 2 g/L of bovine serum albumin (Sigma, Fraction V). The reagent solution was stored under the same conditions as in Example 4 and a remaining activity ratio was determined.
As shown in Table 4, it was found that glycerol kinase was stabilized by the sericin hydrolysate.

[Table 4]

**Table 4: Stabilization Effect of Sericin hydrolysate on Glycerol kinase solution**

| | Additive | Addition concentration (g/L) | Remaining ratio (%) |
|---|---|---|---|
| | | | 25°C, 14 days |
| Example 4 | Sericin hydrolysate | 2 | 66 |
| Comparative Example 4 | Bovine serum albumin | 2 | 60 |
| | None | - | 53 |

### Example 5

A reagent solution containing cholesterol oxidase (Toyobo, COO-321) and a sericin hydrolysate as described below was stored at 52°C for 1 hour or at 40°C for 7 days, and a remaining activity ratio (the ratio of activity after storage to activity immediately after preparation) was determined.

### Preparation of Reagent

A reagent having the following composition was prepared.
Potassium phosphate buffer 50mM pH 7.0
Cholesterol oxidase (Toyobo, COO-321) 2,000 U/L
Sericin hydrolysate 4 g/L

### Comparative Example 5

A reagent solution was prepared according to Example 5 except that the sericin hydrolysate was not added or replaced by 4 g/L of bovine serum albumin (Sigma, Fraction V). The reagent solution was stored under the same conditions as in Example 5 and a remaining activity ratio was determined.
As shown in Table 5, it was found that cholesterol oxidase was stabilized by the sericin hydrolysate.

[Table 5]

**Table 5: Stabilization Effect of Sericin hydrolysate on Cholesterol oxidase solution**

| | Additive | Addition concentration (g/L) | Remaining ratio (%) | |
|---|---|---|---|---|
| | | | 52°C, 1 hour | 40°C, 7 days |
| Example 5 | Sericin hydrolysate | 4 | 103 | 100 |
| Comparative Example 5 | Bovine serum albumin | 4 | 93 | 90 |
| | None | - | 90 | 85 |

### Example 6

A reagent solution containing glucose-6-phosphate dehydrogenase (Toyobo, G6D-321) and a sericin hydrolysate as described below was stored at 25°C for 14 days, and a remaining activity ratio (the ratio of activity after storage to activity immediately after preparation) was determined.

### Preparation of Reagent

A reagent having the following composition was prepared.
Potassium phosphate buffer 50mM pH 7.0
Triton X-100 1 g/L
Glucose-6-phosphate dehydrogenase (Toyobo, G6D-321) 3,000 U/L
Sericin hydrolysate 1 g/L

### Comparative Example 6

A reagent solution was prepared according to Example 6 except that the sericin hydrolysate was not added or replaced by 1 g/L of bovine serum albumin (Sigma, Fraction V). The reagent solution was stored under the same conditions as in Example 6 and a remaining activity ratio was determined.
As shown in Table 6, it was found that glucose-6-phosphate dehydrogenase was stabilized by the sericin hydrolysate.

[Table 6]

**Table 6: Stabilization Effect of Sericin hydrolysate on Glucose-6-phosphate dehydrogenase solution**

| | Additive | Addition concentration (g/L) | Remaining ratio (%) |
|---|---|---|---|
| | | | 25°C, 14 days |
| Example 6 | Sericin hydrolysate | 1 | 93 |
| Comparative Example 6 | Bovine serum albumin | 1 | 70 |
| | None | - | 70 |

### Example 7

A reagent solution containing phosphoenolpyruvate carboxylase (Toyobo, PPC-301) and a sericin hydrolysate as described below was stored at 9°C or 25°C for 24 hours, and a remaining activity ratio (the ratio of activity after storage to activity immediately after preparation) was determined.

### Preparation of Reagent

Each reagent having the following composition was prepared.
Tris-HCl 50mM pH 8.0
Triton X-100 1 g/L
Phosphoenolpyruvate carboxylase (Toyobo, PPC-301) 5,000 U/L
Sericin hydrolysate 0.2 to 20 g/L

### Comparative Example 7

A reagent solution was prepared according to Example 7 except that the sericin hydrolysate was not added. The reagent solution was stored under the same conditions as in Example 7 and a remaining activity ratio was determined.
As shown in Table 7, it was found that phosphoenolpyruvate carboxylase was stabilized by the sericin hydrolysate.

[Table 7]

**Table 7: Stabilization Effect of Sericin hydrolysate on Phosphoenolpyruvate carboxylase solution**

| | Additive | Addition concentration (g/L) | Remaining ratio (%) | |
|---|---|---|---|---|
| | | | 9°C, 24 hours | 25°C, 24 hours |
| Example 7 | Sericin hydrolysate | 0.2 | 79 | 42 |
| | | 1 | 82 | 46 |
| | | 5 | 87 | 56 |
| | | 20 | 94 | 70 |
| Comparative Example 7 | None | - | 75 | 39 |

### Example 8

To a solution of 1 g of cholesterol oxidase (Toyobo, COO-321) powder in 10 ml of distilled water was added 0.5 g of a sericin hydrolysate to prepare a cholesterol oxidase solution. Then, the solution was lyophilized to prepare cholesterol oxidase powder containing the sericin hydrolysate. The lyophilized powder was stored at 50°C for 14 days, and a remaining activity ratio (the ratio of activity after storage at 50°C to activity before storage) was determined.

### Comparative Example 8

Cholesterol oxidase powder was prepared according to Example 8 except that the sericin hydrolysate was not added or replaced by 0.5 g of bovine serum albumin (Sigma, Fraction V). The powder was stored under the same conditions as in Example 8 and a remaining activity ratio was determined.
As shown in Table 8, it was found that cholesterol oxidase was stabilized by the sericin hydrolysate.

[Table 8]

**Table 8: Stabilization Effect of Sericin hydrolysate on Cholesterol oxidase powder**

| | Additive | Remaining ratio (%) |
|---|---|---|
| | | 50°C, 14 days |
| Example 8 | Sericin hydrolysate | 85 |
| Comparative Example 8 | Bovine serum albumin | 77 |
| | None | 51 |

### Example 9

To a solution of 1 g of PQQ-dependent glucose dehydrogenase (Toyobo, GLD-321) powder in 10 ml of distilled water was added 0.5 g of a sericin hydrolysate to prepare a PQQ-dependent glucose dehydrogenase solution. Then, the solution was lyophilized to prepare PQQ-dependent glucose dehydrogenase powder containing the sericin hydrolysate. The lyophilized powder was stored at 37°C for 21 days, and a remaining activity ratio (the ratio of activity after storage at 37°C to activity before storage) was determined.

### Comparative Example 9

PQQ-dependent glucose dehydrogenase powder was prepared according to Example 9 except that the sericin hydrolysate was not added or replaced by 0.5 g of bovine serum albumin (Sigma, Fraction V). The powder was stored under the same conditions as in Example 9 and a remaining activity ratio was determined.
As shown in Table 9, it was found that PQQ-dependent glucose dehydrogenase was stabilized by the sericin hydrolysate.

[Table 9]

**Table 9: Stabilization Effect of Sericin hydrolysate on PQQ-dependent glucose dehydrogenase powder**

| | Additive | Remaining ratio (%) |
|---|---|---|
| | | 37°C, 21 days |
| Example 9 | Sericin hydrolysate | 89 |
| Comparative Example 9 | Bovine serum albumin | 73 |
| | None | 65 |

### Example 10

To a solution of 1 g of creatinine amidohydrolase (Toyobo, CNH-211) powder in 10 ml of distilled water was added 0.5 g of a sericin hydrolysate to prepare a creatinine amidohydrolase solution. Then, the solution was lyophilized to prepare creatinine amidohydrolase powder containing the sericin hydrolysate. The lyophilized powder was stored at 37°C for 14 days, and a remaining activity ratio (the ratio of activity after storage at 37°C to activity before storage) was determined.

### Comparative Example 10

Creatinine amidohydrolase powder was prepared according to Example 10 except that the sericin hydrolysate was not contained. The powder was stored under the same conditions as in Example 10 and a remaining activity ratio was determined.
As shown in Table 10, it was found that creatinine amidohydrolase was stabilized by the sericin hydrolysate.

[Table 10]

**Table 10: Stabilization Effect of Sericin hydrolysate on Creatinine amidohydrolase powder**

| | Additive | Remaining ratio (%) |
|---|---|---|
| | | 37°C, 14 days |
| Example 10 | Sericin hydrolysate | 96 |
| Comparative Example 10 | None | 87 |

### Examples 11 to 16 and Comparative Example 11

Stabilization of horseradish peroxidase-labeled antibody

### Comparative Example 11

A 8000-fold dilution of peroxidase-labeled anti-mouse IgG (Amersham Biosciences) with 20mM MOPS buffer (pH 7.0) containing 5 g/L of bovine serum albumin (Nacalai Tesque) was filtrated through a gamma-ray-sterilized 0.22-µm filter, and then stored in a gamma-ray-sterilized polypropylene container at 4°C or 25°C for 3 days.

### Example 11

A 8000-fold dilution of peroxidase-labeled anti-mouse IgG (Amersham Biosciences) with 20mM MOPS buffer (pH 7.0) containing 5 g/L of a sericin hydrolysate was filtrated through a gamma-ray-sterilized 0.22-µm filter, and then stored in a gamma-ray-sterilized polypropylene container at 4°C or 25°C for 3 days.

### Example 12

A sericin hydrolysate was dissolved in distilled water at a concentration of 100 g/L, and then autoclaved at 121°C for 20 minutes. A 8000-fold dilution of peroxidase-labeled anti-mouse IgG (Amersham Biosciences) with 20mM MOPS buffer (pH 7.0) containing 5 g/L of the autoclaved sericin hydrolysate was filtrated through a gamma-ray-sterilized 0.22-µm filter, and then stored in a gamma-ray-sterilized polypropylene container at 4°C or 25°C for 3 days.

### Example 13

A 8000-fold dilution of peroxidase-labeled anti-mouse IgG (Amersham Biosciences) with 20mM MOPS buffer (pH 7.0) containing 5 g/L of a sericin hydrolysate and 0.2 g/L of 4-aminoantipyrine (Nacalai Tesque) was filtrated through a gamma-ray-sterilized 0.22-µm filter, and then stored in a gamma-ray-sterilized polypropylene container at 4°C or 25°C for 3 days.

### Example 14

A 8000-fold dilution of peroxidase-labeled anti-mouse IgG (Amersham Biosciences) with 20mM MOPS buffer (pH 6.5) containing 5 g/L of a sericin hydrolysate and 0.2 g/L of 4-aminoantipyrine (Nacalai Tesque) was filtrated through a gamma-ray-sterilized 0.22-µm filter, and then stored in a gamma-ray-sterilized polypropylene container at 4°C or 25°C for 3 days.

### Example 15

### Preparation of antibody solid phase plate reagent

A 10 µg/ml dilution of a goat anti-mouse Fc antibody (Sigma) with 50mM carbonate buffer (pH 9.6) was dispensed into wells of an ELISA plate (Sumitomo Bakelite) at 50 µL/well. The plate was left to stand at room temperature for 1 hour to allow the antibody to bind to the plate. Then, the liquid in the wells was thoroughly removed. Then, a 4-fold dilution of Block Ace (Dainippon Pharmaceutical) with distilled water was dispensed to the wells of the plate at 300 µL/well. The plate was left to stand at room temperature for 1 hour for blocking. The liquid in the wells was thoroughly removed. A 10mM phosphate buffered saline (pH 7.2) containing 0.5 g/L of Tween 20 was dispensed into the wells at 300 µL/well and then removed from the wells, and this procedure was repeated three times, whereby the plate was washed (hereinafter, referred to as PBS-T washing). Then, the plate was used for measurement.

### Example 16

### Measurement of mouse IgG

Mouse IgG (Chemicon) was diluted with a phosphate-buffered saline containing 0.1% BSA to prepare a sample containing 64, 128 or 256 ng/ml of mouse IgG. The buffer used for dilution was used as a 0 ng/ml sample. Then, each sample was dispensed into the wells of the ELISA plate at 50 µL/well (N=2), and the plate was left to stand at room temperature for 1 hour for reaction. After PBS-T washing of the plate, each labeled antibody solution prepared in Comparative Example and Examples was dispensed into the wells at 50 µL/well. The plate was left to stand at room temperature for 1 hour for reaction. After PBS-T washing of the plate, 50 µL of TMB+ (Dako) was added into each well as an enzymatic reaction mixture, and the plate was left to stand at room temperature under a dark condition for 10 minutes for reaction. The enzymatic reaction was terminated by adding 50 µL of 1N sulfuric acid into each well and then stirring the mixtures. Then, the plate was subjected to measurement of O.D. at 450 nm to 650 nm using a microplate reader. The absorbance was increased in a mouse IgG concentration/dose-dependent manner.
Using results obtained by ELISA, a difference in absorbance between the 256 ng/ml sample and the 0 ng/ml sample was calculated. The ratio of an absorbance difference obtained from a test to an absorbance difference obtained from the corresponding test using the labeled antibody stored at 4°C was calculated, and the cubic root of the ratio was extracted. Thus, the remaining activity ratio per day of the antibody was determined. Results are shown in Table 11. When the labeled antibody solutions containing a sericin hydrolysate of Examples were used, higher remaining activity ratios were observed as compared with Comparative Example using bovine serum albumin, and thus the stabilization effect of a sericin hydrolysate was found. The stabilization effect was not lost even after a sericin hydrolysate was autoclaved. The remaining activity ratio could be further increased by the coexistence of a sericin hydrolysate and a substance which could act as a substrate for peroxidase.

[Table 11]

**Table 11: Stabilization Effect of Sericin hydrolysate on Horseradish peroxidase-labeled antibody**

| | Comparative Example 11 | Example 11 | Example 12 | Example 13 | Example 14 |
|---|---|---|---|---|---|
| Remaining activity ratio per day (%) | 85 | 96 | 96 | 99 | 99 |

### Examples 17 to 21 and Comparative Examples 12 and 13

Stabilization of alkaline phosphatase-labeled antibody

### (1) Example 17

### Preparation of alkaline phosphates-labeled antibody

An alkaline phosphatase-labeled antibody was prepared using 200 µg of mouse anti-CEA monoclonal antibody clone 5905 (Medix Biochemica) and a labeling kit for binding with an amino group of an antibody (Dojindo, LK12) according to the instruction attached to the labeling kit. Briefly, 100 µL of the washing buffer attached to the kit was placed in the attached filtration tube and lightly mixed using a pipette. The tube was centrifuged at 8000 x g for 10 minutes. After further 100 µL of the washing buffer was added, the tube was centrifuged once more under the same conditions. In the NH2-Reactive ALP attached to the kit, 10 µL of the attached reaction buffer was dissolved by pipetting. The total amount of the solution was added onto the membrane of the filtration tube on which the antibody had been concentrated, and then mixed with the antibody on the membrane by pipetting. The tube was left to stand at 37°C for 2 hours. Then, 190 µL of the storage buffer attached to the kit was added to the tube, and 200 µL of a labeled antibody was recovered by pipetting, which was used as an alkaline phosphatase-labeled anti-CEA monoclonal antibody.

### (2) Comparative Example 12

A 8000-fold dilution of the alkaline phosphatase-labeled anti-CEA monoclonal antibody with a 0.1M Tris-HCl buffer (pH 7.4) containing 1mM magnesium chloride, 0.1mM zinc chloride and 1 g/L of sodium azide was filtrated through a gamma-ray-sterilized 0.22-µm filter, and then stored in a gamma-ray-sterilized polypropylene container at 4°C or 25°C for 3 days.

### (3) Comparative Example 13

A 8000-fold dilution of the alkaline phosphatase-labeled anti-CEA monoclonal antibody with a 0.1M Tris-HCl buffer (pH 7.4) containing 20 g/L of bovine serum albumin (Nacalai Tesque), 1mM magnesium chloride, 0.1mM zinc chloride and 1 g/L of sodium azide was filtrated through a gamma-ray-sterilized 0.22-µm filter, and then stored in a gamma-ray-sterilized polypropylene container at 4°C or 25°C for 3 days.

### (4) Example 18

A 8000-fold dilution of the alkaline phosphatase-labeled anti-CEA monoclonal antibody with a 0.1M Tris-HCl buffer (pH 7.4) containing 20 g/L of a sericin hydrolysate, 1mM magnesium chloride, 0.1mM zinc chloride and 1 g/L of sodium azide was filtrated through a gamma-ray-sterilized 0.22-µm filter, and then stored in a gamma-ray-sterilized polypropylene container at 4°C or 25°C for 3 days.

### (5) Example 19

A sericin hydrolysate was dissolved in distilled water at a concentration of 100 g/L, and then autoclaved at 121°C for 20 minutes. A 8000-fold dilution of the alkaline phosphatase-labeled anti-CEA monoclonal antibody with a 0.1M Tris-HCl buffer (pH 7.4) containing 20 g/L, of the autoclaved sericin hydrolysate, 1mM magnesium chloride, 0.1mM zinc chloride and 1 g/L of sodium azide was filtrated through a gamma-ray-sterilized 0.22-µm filter, and then stored in a gamma-ray-sterilized polypropylene container at 4°C or 25°C for 3 days.

### (6) Example 20

### Preparation of anti-CEA antibody solid phase plate reagent

A 10 µg/ml dilution of mouse anti-CEA monoclonal antibody clone 5909 (Medix Biochemica) with 50mM carbonate buffer (pH 9.6) was dispensed into wells of a black polystyrene assay plate (Corning) at 50 µL/well. The plate was left to stand at room temperature for 1 hour to allow the antibody to bind to the plate. Then, the liquid in the wells was thoroughly removed. Then, a 4-fold dilution of Block Ace (Dainippon Pharmaceutical) with distilled water was dispensed to the wells of the plate at 300 µL/well. The plate was left to stand at room temperature for 1 hour for blocking. The liquid in the wells was thoroughly removed. A 10mM Tris-buffered saline (pH 7.5) containing 0.5 g/L of Tween 20 was dispensed into the wells at 300 µL/well and then removed from the wells, and this procedure was repeated three times, whereby the plate was washed (hereinafter, referred to as TBS-T washing). Then, the plate was used for measurement.

### (7) Example 21

### Measurement of CEA

### Measurement of mouse IgG

CEA antigen was diluted with a phosphate-buffered saline containing 0.1% BSA to prepare a sample containing 5, 20 or 80 ng/ml of CEA. The buffer used for dilution was used as a 0 ng/ml sample. Then, each sample was dispensed into the wells of the ELISA plate at 50 µL/well (N=2), and the plate was left to stand at 37°C for 1 hour for reaction. After TBS-T washing of the plate, each labeled antibody solution prepared in Comparative Example 3 and Example 8 was dispensed into the wells at 50 µL/well. The plate was left to stand at 37°C for 1 hour for reaction. After TBS-T washing of the plate, 50 µL of APS-5 (Lumigen) was added into each well as an enzymatic reaction mixture. The plate was left to stand at 37°C for 20 minutes for reaction. Then, the plate was subjected to measurement of a luminescence signal using Luminoscan (Labsystems). The luminescence intensity was increased in a CEA concentration/dose-dependent manner.
Using the obtained results, a difference in luminescence intensity between the 80 ng/ml sample and the 0 ng/ml sample was calculated. The ratio of a luminescence intensity difference obtained from a test to a luminescence intensity difference obtained from the corresponding test using the labeled antibody stored at 4°C was calculated, and the cubic root of the ratio was extracted. Thus, the remaining activity ratio per day of the antibody was determined. Results are shown in Table 12. As seen from Comparative Examples, the addition of bovine serum albumin, which was a conventional technique, increased the stability of the alkaline phosphatase-labeled antibody. Surprisingly, when the labeled antibody solutions containing a sericin hydrolysate of Examples were used, higher remaining activity ratios were observed, and thus it was found that a sericin hydrolysate had an improved stabilization effect on an alkaline phosphatase-labeled antibody. The stabilization effect was not lost even after a sericin hydrolysate was autoclaved.

[Table 12]

**Table 12: Stabilization Effect of Sericin hydrolysate on Alkaline phosphatase-labeled antibody**

| | Comparative Example 12 | Comparative Example 13 | Example 18 | Example 19 |
|---|---|---|---|---|
| Remaining activity ratio per day (%) | 68 | 94 | 99 | 99 |

### Industrial Applicability

According to the present invention, it is possible to enhance stabilization of a biological molecule such as an enzyme or a labeled antibody, and thereby, the efficacy of a composition comprising the biological molecule can be maintained over a long period of time. In particular, the present invention is excellently applied to diagnostic agents used in the clinical laboratory test field. Thus, the present invention greatly contributes to the industrial world.

## Claims

1. A method for stabilizing a biological molecule, which comprises allowing the following (a) and (b) to coexist with each other:
(a) a biological molecule; and
(b) sericin and/or a hydrolysate or equivalent thereof.

2. The method according to claim 1, wherein the biological molecule is present in a solution.

3. The method according to claim 1, wherein the biological molecule is present in a lyophilized composition.

4. The method according to claim 1, wherein the sericin and/or a hydrolysate thereof is derived from naturally-occurring sericin extracted from cocoon filaments or raw silk.

5. The method according to claim 1, wherein the equivalent of sericin is obtained by a genetic engineering technique.

6. The method according to claim 1, wherein the biological molecule is a protein.

7. The method according to claim 1, wherein the biological molecule is an enzyme.

8. The method according to claim 1, wherein the biological molecule is a labeled antibody.

9. A composition comprising a stabilized biological molecule, wherein the following (a) and (b) coexist with each other in the composition:
(a) a biological molecule; and
(b) sericin and/or a hydrolysate or equivalent thereof.

10. The liquid composition according to claim 9, wherein the biological molecule is present in a solution.

11. The composition according to claim 9, wherein the biological molecule is present in a lyophilized composition.

12. The composition according to claim 9, wherein the sericin and/or a hydrolysate thereof is derived from naturally-occurring sericin extracted from cocoon filaments or raw silk.

13. The composition according to claim 9, wherein the equivalent of sericin is obtained by a genetic engineering technique.

14. The composition according to claim 9, wherein the biological molecule is a protein.

15. The composition according to claim 9, wherein the biological molecule is an enzyme.

16. The composition according to claim 9, wherein the biological molecule is a labeled antibody.

17. A method for producing a composition comprising a stabilized biological molecule, which comprises a step of allowing sericin and/or a hydrolysate or equivalent thereof to coexist with a biological molecule.

18. A composition for stabilizing a biological molecule, comprising sericin and/or a hydrolysate or equivalent thereof.

19. A kit for diagnosis which comprises the composition comprising a stabilized biological molecule according to claim 9.

20. A biosensor which comprises the composition comprising a stabilized biological molecule according to claim 9.
